# EUROPÄISCHE PATENTSCHRIFT

(11) **EP 3 982 853 B1**
(45) Veröffentlichungstag und Bekanntmachung des Hinweises auf die Patenterteilung: **13.03.2024**
(21) Anmeldenummer: 20733723.9
(22) Anmeldetag: 15.06.2020
(51) Int. Cl.: A61B 17/70

(54) **TEILWEISE BLOCKIERTE PEDIKELSCHRAUBE**
PARTIALLY BLOCKED PEDICAL SCREW
VIS PÉDICULAIRE BLOQUÉE EN PARTIE

(30) Priorität: 17.06.2019 DE 102019116374
(43) Veröffentlichungstag der Anmeldung: 20.04.2022
(73) Patentinhaber: AESCULAP AG, 78532 Tuttlingen (DE)
(72) Erfinder: WENZEL, Rudolf, 66620 Nonnweiler-Primstal (DE); BACKES, Hermann, 66620 Nonnweiler-Primstal (DE)
(74) Vertreter: Winter, Brandl - Partnerschaft mbB
(86) Internationale Anmeldenummer: PCT/EP2020/066476
(87) Internationale Veröffentlichungsnummer: WO 2020/254243

(56) Entgegenhaltungen:
- US-A1- 2011 009 911
- US-A1- 2013 023 935
- US-A1- 2014 343 617
- US-A1- 2015 282 844

## Beschreibung

Die vorliegende Offenbarung betrifft eine teilweise blockierte Pedikelschraube (kurz: Schraube) der polyaxialen Bauart mit einem Schraubenschaft und einem damit stoffeinstückig verbundenen Schraubenkopf, welcher drehbar und/oder verschwenkbar in einer Aufnahmehülse (Tulpe) gelagert bzw. aufgenommen ist.

### Hintergrund der Erfindung

Pedikelschrauben, insbesondere der Polyaxialbauart, werden bei chirurgischen Eingriffen an der Wirbelsäule verwendet, um mehrere Wirbel zueinander lagebestimmt festzulegen. Die Schrauben werden dazu jeweils in Pedikel eines Wirbels eingeschraubt und mittels einem in die Schraubenköpfe bzw. in die damit verbundenen Tulpen eingesetztem Verbindungsstab verbunden. Je nach Einsatzzweck kommen dabei Schrauben mit unterschiedlichen Freiheitsgraden bezüglich einer Relativbewegung zwischen einer Tulpe und einem Schraubenschaft zum Einsatz. Beispielsweise werden zur Derotation von deformierten Wirbelsäulen Schrauben vorgesehen, bei welchen eine Schwenkbewegung zwischen der Tulpe und dem Schraubenschaft in medial-lateraler Richtung blockiert ist (d.h. in seitlicher Richtung bezüglich einer Wirbelsäule bzw. des zugehörigen menschlichen Körpers), in kranial-kaudaler Richtung (d.h. in Längsrichtung oder Höhenrichtung bezüglich der Wirbelsäule) jedoch zur Positionierung während der Operation zugelassen wird. Dies wird dadurch erreicht, indem der Schraubenkopf unrund, beispielsweise mit seitlichen Abflachungen, ausgebildet ist und ein in die Tulpe eingesetztes Inlay oder Insert zur axialen Druckbeaufschlagung des Schraubenkopfs für ein Fixieren der Relativlage zwischen Schraube und Tulpe mit entsprechenden seitlichen Innenflächen ausgebildet ist, die mit den Abflachungen am Schraubenkopf in Auflage kommen.

Derartige Pedikelschrauben werden mittels eines speziellen Instruments oder Instrumentensatzes implantiert, welches/welcher in der Regel für eine Anzahl von unterschiedlichen Pedikelschrauben standardisiert ist.

### Stand der Technik

Aus dem Stand der Technik, beispielsweise aus US 2010/0305621 A1 und US 9 138 261 B2, ist jeweils eine polyaxiale Pedikelschraube bekannt, deren runder, in einem Aufnahmekörper eingesetzter Kopf an zwei einander gegenüberliegenden Seiten zumindest teilweise abgeflacht ist, sodass eine proximal des Kopfes angeordnete Buchse (Inlay/Insert zum axialen Einsetzen in eine Tuplpe) mit zwei ohrenförmigen axialen Vorsprüngen, welche flache Innenflächen ausbilden, mit diesen Innenflächen an den abgeflachten Seiten des Kopfs geführt sind und eine Verschwenkung des Schraubenschafts und -kopfs gegenüber der Buchse nur in einer Ebene parallel zu den flachen Innenflächen erlauben. Ein Teil des Kopfes der Pedikelschraube kann über die abgeflachten Seiten hinaus schraubenradial vorstehen, um eine zusätzliche schraubendiametrale Abstützung des Schraubenkopfs an dem Aufnahmekörper ermöglichen. Dies begrenzt jedoch einen erreichbaren Verschwenkungswinkel und verkleinert die Führungsfläche. Ferner sind die Flansche geringfügig elastisch deformierbar. Demgemäß können hohe Querkräfte ggf. nicht ausreichend abgestützt werden.

US 8 048 133 B2 offenbart eine polyaxiale Pedikelschraube, deren runder Kopf an zwei einander schraubendiametral gegenüberliegenden Seiten bearbeitet ist, um einen schraubendiametral verlaufenden Zylinder zu bilden, welcher proximal mit einer entsprechenden Fläche eines Druckelements (Inserts/Inlays) in Anlage kommt und zusammen mit dieser Fläche eine Art Gleitlager bildet, welches Drehungen um eine Zylinderachse erlaubt und Drehungen/Schwenkbewegungen um andere Achsen blockiert. Dieses Blockieren einer Drehung oder Verschwenkung um andere Achsen ist jedoch bei hohen Querkräften voraussichtlich nicht ausreichend stabil und führt zu einem schnellen Ausleiern oder Abnutzen der Verbindung.

Ein weiteres Dokument, US 5 989 254 A, zeigt eine polyaxiale Pedikelschraube mit einem kugeligen Kopf, in welchem eine zylindrische Sattelfläche bzw. ein Querschlitz mit sattelförmig längsgekrümmten Schlitzgrund zur Aufnahme einer Verbindungsstange, mittels welcher mehrere Pedikelschrauben verbindbar sind, ausgespart ist. Im Fall, dass eine Tulpe über den Schraubenkopf der Pedikelschraube gesetzt und eine Verbindungsstange in die Tulpe quereingelegt ist, wälzt sich die Sattelfläche bei einer Verschwenkung des Schraubenkopfs in der Tulpe um eine schraubendiametral verlaufende Achse an der Verbindungsstange ab. Querkräfte werden hauptsächlich über eine Außenfläche der Verbindungsstange und Seitenflächen der Aussparung für die Sattelfläche, also über einen relativ kurzen Linienkontakt, übertragen. Diese Konstruktion ist gegenüber hohen Querkräften voraussichtlich nicht ausreichend stabil.

Ferner ist aus US 2015/282844 A1 ein Knochenanker mit den Merkmalen des Oberbegriffs des Anspruchs 1 bekannt. Der untere Bereich des Gehäuses definiert Passelemente, die mit Eingriffselementen an der Knochenschraube zusammenpassen, um die Bewegung des Gehäuses relativ zur Knochenschraube auf eine einzige Ebene zu begrenzen. Die Schraube hat einen im Wesentlichen kugelförmigen Kopf, welcher gegenüberliegende ebenen Flächen und Verlängerungen aufweist, wobei die Verlängerungen der kugelförmigen Oberfläche des Kopfes folgen und die ebenen Oberflächen durch Entfernen von Material unter den Verlängerungen gebildet sind.

US 2013/023935 A1offenbart ein Wirbelsäulenstabilisierungssystem zur chirurgischen Implantation in die Wirbelsäule mit einem Knochenbefestigungselement, das einen Schaftteil und einen Kopfteil mit einem rechteckig geformten unteren Teil und einem gekrümmten oberen Teil aufweist. Außerdem ist ein Gehäuse mit einer durch Seitenwände definierten Aussparung und einer Öffnung zur Aufnahme des Kopfteils des Knochenbefestigungselements vorgesehen. Das Knochenbefestigungselement ist in einer einzigen Ebene in Bezug auf das Gehäuse und das Sitzelement beweglich.

Zusammenfassend lässt sich sagen, dass bekannte Lösungen in vielen Anwendungssituationen nicht in der Lage sind, ausreichend hohe Querbelastungen bei einem Blockieren von Querverschwenkungen aufzunehmen (das heißt, die erlaubten Querkräfte sind zu niedrig). Ferner weisen diese Lösungen zu viele Einzelteile der polyaxialen Pedikelschraube auf und/oder enthalten Gelenke und sind somit aufwändig zu fertigen und zu montieren. Insbesondere ist häufig die Innengeometrie proximal in den Schraubenkopf eingesetzten des Inserts oder Einsetz- bzw. Haltestücks innerhalb der Tulpe aufwändig. Dies erhöht u.a. die Fertigungskosten. Ggf. ist für eine Implantation dieser Schrauben zudem ein eigenes Instrumentarium notwendig, was zugehörige Fertigungskosten aufgrund geringerer Stückzahlen weiter erhöht.

### Kurzbeschreibung der Erfindung

Der vorliegenden Erfindung liegt eine Aufgabe zugrunde, Nachteile des Stands der Technik zu reduzieren oder zu vermeiden. Insbesondere soll eine einfache, stabile Pedikelschraube mit einem Schraubenkopf bereitgestellt werden, der innerhalb einer Aufnahmehülse bzw. Tulpe in einer (einzigen) schraubendiametralen Richtung (insbesondere kranial-kaudal) verschwenkbar ist und in einer anderen schraubendiametralen Richtung (insbesondere medial-lateral) auch bei hohen Querkräften/Seitenlasten von mindestens 500N lagefestgelegt ist. Ferner sollen mit der vorliegenden Pedikelschraube insbesondere das Instrumentarium und Madenschrauben zum Fixieren der Schraube, welche vorzugsweise mehrfach gelöst und angezogen werden können sollen, gleicher Systeme verwendbar sein. In anderen Worten sollen die grundsätzlichen Funktionen und die Außengeometrie der "normalen" (systemgleichen) polyaxialen Pedikelschraube beibehalten werden.

Zusammenfassung der Erfindung.

Die der Erfindung zugrunde liegende Aufgabe wird durch eine Pedikelschraube mit den Merkmalen des Anspruchs 1 gelöst. Vorteilhafte Ausgestaltungen der Erfindung sind Gegenstand der Unteransprüche und werden später genauer beschrieben.

Der Grundgedanke der vorliegenden Erfindung liegt im Wesentlichen darin, bei der erfindungsgemäßen Pedikelschraube zusätzliche Stütz-/Lager-/Führungsbereiche zwischen einer Aufnahmehülse oder Tulpe und einem darin gelagerten Schraubenkopf bereitzustellen, welche bei einer auftretenden Querlast eine Querverschwenkung der Aufnahmehülse oder Tulpe relativ zu einem Schraubenschaft, der distal mit dem Schraubenkopf integral verbunden ist, verhindern. Andererseits soll dabei durch diese zusätzlichen Stützbereiche die Beweglichkeit des Schraubenkopfs relativ zu der Aufnahmehülse oder Tulpe der Pedikelschraube um eine Schwenkachse nicht behindert werden. Erfindungsgemäß werden an dem sphärischen Schraubenkopf darüber hinausragende Fortsätze bereitgestellt, welche diese zusätzlichen Stützflächen ausbilden und welche unmittelbar mit der Aufnahmehülse in Stütz-/Lager-/Führungskontakt gebracht werden können oder sind. Auf diese Weise wird der Schraubenkopf nicht nur, wie bei polyaxialen Pedikelschrauben üblich, über einen Stützbereich zwischen dem Schraubenkopf und einer proximal des Schraubenkopfs in der Aufnahmehülse oder Tulpe angeordneten Einsetzhülse/Inlay/Insert, welches dazu dient gegen den Schraubenkopf zu pressen und dadurch dessen Relativlage in der Aufnahmehülse/Tulpe festzulegen, gegen die Querverschwenkung gesichert. Stattdessen sind die Fortsätze zur Ausbildung eines zusätzlichen Stützbereichs mit der Aufnahmehülse/Tulpe bereitgestellt, um eine solche Querverschwenkung zu verhindern. Vorzugsweise können zudem weitere Stützbereiche bereitgestellt werden, welche in unterschiedliche Richtungen ausgerichtet sind, wie beispielsweise eine quer zu der Schraubenlängsachse verlaufende Zylinderfläche, um die Querbelastung stets möglichst optimal aufzunehmen.

Genauer ausgedrückt, wird die der Erfindung zugrunde liegende Aufgabe durch eine Pedikelschraube der Polyaxialbauart gelöst, welche einen Schraubenschaft hat, an dessen proximalen Ende ein Schraubenkopf mit im Wesentlichen sphärischer Grundform einstückig ausgebildet ist, der in einer Aufnahmehülse oder Tulpe gelagert ist, in der eine Einsetzhülse eingesetzt ist, die zur Festlegung einer Relativ-Schwenkposition zwischen der Aufnahmehülse oder Tulpe und dem Schraubenschaft gegen den Schraubenkopf andrückbar ist. Zwei einander diametral gegenüberliegende Schwenkführungs- oder Schwenkbeschränkungseinheiten sind bereitgestellt, die jeweils zumindest einen radial sowie in Schraubenlängsrichtung über die sphärische Grundform des Schraubenkopfs hinausragenden Fortsatz aufweisen, wobei die Fortsätze an ihren jeweiligen, vorzugsweise teilzylinderförmigen, radialen Außenflächen Anschläge bilden. Die Fortsätze sind derart positioniert, dass sie eine Relativ-Verschwenkung von Schraubenschaft und Tulpe oder Aufnahmehülse nur in der Schwenkebene erlauben und dass sie bei einer Relativ-Verschwenkung in einer anderen Schwenkebene (einer Querverschwenkung) gegen eine radial innere Umfangsseite der Tulpe oder Aufnahmehülse stützend anschlagen. Durch die auf diese Weise erzeugten Stützbereiche/Anschläge zwischen dem Schraubenkopf und der Aufnahmehülse/Tulpe kann erreicht werden, dass sehr hohe Querlasten (mindestens 500N) übertragbar sind und Querverschwenkungen effektiv blockiert werden können. Es wird folglich eine hohe Seitenstabilität durch dicke Fortsätze oder Führungslaschen erreicht.

Ferner sind keine Änderungen an der Außengeometrie der Gesamtschraube notwendig, weshalb für eine Implantation der Pedikel- oder Knochenschraube systemgleiche Standardinstrumente verwendet werden können. Entsprechend sind für eine solche Pedikelschraube nur schraubeninterne Änderungen der Konstruktion im Vergleich mit einer systemgleichen Standardschraube vorgenommen, ohne dass eine komplexe Innengeometrie am Insert notwendig wäre. Die Einsetzhülse (Insert/Inlay) kann geometrisch im Wesentlichen unverändert oder nur mit relativ geringfügigen Änderungen sowohl für eine Uniplanarschraube als auch für eine Polyaxialschraube verwendet werden, weshalb ggf. höhere Stückzahlen und niedrigere Kosten erzielt werden können. Ferner muss der Montageprozess minimal oder sogar gar nicht verändert werden, weshalb keine Umstellung der Montage notwendig ist. Auch die Anzahl der benötigten Teile (Aufnahmehülse/Tulpe/Body, Einsetzhülse/Insert/Inlay und Schraubenkopf und -schaft) ist gering. Weiterhin haben die einzelnen Komponenten, das heißt, der Schraubenkopf, die Einsetzhülse (Inlay/Insert) und die Aufnahmehülse (Tulpe), eine einfache Form, wodurch die Pedikelschraube einfach zu fertigen ist.

Vorzugsweise bilden die Fortsätze proximal teilweise zylinderförmige Lagerflächen, welche die Relativ-Verschwenkung des Schraubenschafts und der Tulpe oder Aufnahmehülse in der Schwenkebene führen und welche in proximaler Richtung an stirnseitigen oder distalen, entsprechend teilzylindrisch ausgebildeten Rücksprüngen der Einsetzhülse geführt sind, um nach Art eines Gleitlagers zusammenzuwirken. Eine so gebildete Wirkflächenpaarung dient sowohl zur gleichmäßigen, möglichst reibungsarmen Führung der Verschwenkung um die Schwenkachse als auch zum Abstützen oder Blockieren der Querschwenkung, insbesondere um eine Achse quer zu der Schwenkachse. Da die Fortsätze einander diametral gegenüberliegen, entsteht zudem zwischen den zumindest zwei Fortsätzen bzw. den entsprechenden Lagerflächen ein Hebel, über welchen auf die Schraube wirkende Querkräfte besonders gut übertragen werden können. Demgemäß kann eine Verschwenkung des Schraubenkopfes relativ zu der Aufnahmehülse/Tulpe um eine Achse quer zu der Schwenkachse effektiv blockiert werden.

Es hat sich als zweckmäßig erwiesen, dass die Tulpe oder Aufnahmehülse an einer Innenumfangsfläche zumindest zwei einander diametral gegenüberliegende Aussparungen aufweist, welche ausreichend groß dimensioniert sind, dass die Fortsätze in jeder Schwenkposition darin aufgenommen sind. Ohne diese Aussparungen wäre aufgrund der Fortsätze die Verschwenkung des Schraubenkopfs relativ zu der Aufnahmehülse/Tulpe nicht oder nur geringfügig möglich. Demgemäß wird die Verschwenkbarkeit des Schraubenkopfes in der Aufnahmehülse/Tulpe insbesondere durch die Aussparungen gewährleistet. Bevorzugter Weise sind die Aussparungen und die Fortsätze derart dimensioniert und positioniert, dass dir Fortsätze in jeder Schwenkposition an der radial inneren Umfangsseite der Tulpe oder Aufnahmehülse anschlagen können. Dies kann beispielsweise erzielt werden, indem die Aussparungen im Wesentlichen rechteckig sind. Dadurch wird in der Aufnahmehülse/Tulpe eine große, insbesondere eine sich in Schraubenlängsrichtung erstreckende Anschlags- oder Anlagefläche für die Fortsätze des Schraubenkopfs bereitgestellt, wodurch hohe auftretende Querkräfte (mindestens 500N) unmittelbar und stabil auf die Aufnahmehülse/Tulpe übertragen werden können. Demgemäß kann über eine Wechselwirkung zwischen den Fortsätzen des Schraubenkopfes und der Aufnahmehülse sowie, wie vorstehend beschrieben, über eine Wechselwirkung zwischen den Fortsätzen des Schraubenkopfes und der Einsetzhülse, eine Verschwenkung um eine Achse quer zu der Schwenkachse effektiv unterbunden werden.

Es ist von Vorteil, wenn durch die Fortsätze eine Verschwenkung des Schraubenkopfs in einer medial-lateralen Richtung blockiert wird und eine Verschwenkung in einer kranial-kaudalen Richtung zugelassen wird. Dies ist insbesondere auf dem Einsatzgebiet der Wirbelsäulenderotation vorteilhaft. Konstruktiv wird dies dadurch erreicht, dass die Aufnahmehülse oder Tulpe und vorzugsweise die Einsetzhülse in Umfangsrichtung um 90° zu den im zusammengebauten Zustand darin angeordneten Fortsätzen versetzt vorzugsweise U-förmige Schlitze zur Aufnahme einer Verbindungsstange aufweist, durch welche mehrere Pedikelschrauben miteinander verbindbar sind. Grundsätzlich kann dies für andere Anwendungszwecke auch umgekehrt vorgesehen werden. Das heißt, es sind verschiedene Ausrichtungen des Schraubenkopfs zu der Aufnahmehülse/Tulpe möglich. Ferner soll bevorzugter Weise bei der Pedikelschraube eine Verschwenkung des Schraubenschafts relativ zu der Aufnahmehülse oder Tulpe in der einen Schwenkebene von zumindest +/- 22°, vorzugsweise zumindest +/- 30°, möglich sein. Auch dieser Bereich orientiert sich an für die Wirbelsäulenderotation vorteilhaften Werten.

Nach einer vorteilhaften Ausgestaltung der Erfindung ist ein Durchmesser der proximalen Lagerflächen der Fortsätze kleiner als ein Durchmesser der sphärische Grundform des Schraubenkopfs. Das heißt, die Fortsätze sind im Wesentlichen halbmondförmig oder ohrenartig an dem sphärischen Grundkörper des Schraubenkopfes (insbesondere in der Nähe von dessen schraubendiametralem Außenumfang) angeordnet, wobei ein runder Teil des Halbmonds oder der Ohren in proximaler Richtung ausgerichtet ist. Der Durchmesser der sphärischen Grundform des Schraubenkopfs, insbesondere eine Außenkontur des gesamten Schraubenkopfs, ist in diesem Fall vorzugsweise kleiner oder gleich einem Innendurchmesser der Aufnahmehülse/Tulpe. Dadurch sind an dem Schraubenkopf nur wenige Ecken und Kanten ausgebildet, was dessen Fertigung und ggf. eine Sterilisierung vereinfacht. Ferner ist ein Nachbearbeitungsaufwand der Aufnahmehülse/Tulpe minimiert.

Bevorzugter Weise sind an dem Schraubenkopf zusätzliche Stützlaschen ausgebildet, welche zu den Fortsätzen schraubendiametral nach innen versetzt sind und welche in proximaler Richtung über die Fortsätze hinausragen. Insbesondere sollen diese Stützlaschen proximal teilweise zylinderförmige zweite Lagerflächen bilden, um mit stirnseitigen, entsprechend teilzylindrisch ausgebildeten Schlitzen der Einsetzhülse nach Art eines Gleitlagers zusammenzuwirken. Ein Durchmesser der zweiten Lagerflächen ist größer als der der vorstehend beschriebenen ersten Lagerflächen der Fortsätze. Dadurch können weitere Flächen zur Führung und Kraftübertragung bei der vorgesehenen Schwenkbewegung bereitgestellt werden und werden Querbelastungen besser abgestützt.

Beispielsweise kann es von Vorteil sein, wenn die Stützlaschen schraubenradial außen und ggf. auch innen flach sind, um sich an korrespondierenden Flächen der Schlitze abzustützen, um die Verschwenkung in der anderen Schwenkebene bzw. die Querverschenkung zu blockieren. Das heißt, die Stützlaschen bilden eine schraubendiametrale ebene Fläche an dem Schraubenkopf aus, um einen zusätzlichen radialen Stütz- oder Führungsbereich zum Übertragen von Querlasten bereitzustellen.

Nach einer weiteren vorteilhaften Ausgestaltung der Erfindung können die Fortsätze schraubendiametral nach außen und vorzugsweise proximal über einen Außendurchmesser des sphärischen Grundkörpers des Schraubenkopfs hinausragen. In anderen Worten wird durch diese schraubendiametrale Verbreiterung des Schraubenkopfes ein Hebel zwischen den beiden Fortsätzen bzw. den durch diese ausgebildeten Lagerflächen bereitgestellt. Demgemäß können über die Lagerflächen der Fortsätze des Schraubenkopfs noch größere Querlasten abgestützt werden als nach der vorstehend beschriebenen, ersten vorteilhaften Ausgestaltung.

Es kann ferner eine Außenkontur des gesamten Schraubenkopfs (d.h., des sphärischen Grundkörpers und der darüber schraubendiametral hinausragenden Fortsätze) größer als der Innendurchmesser der Tulpe oder Aufnahmehülse sein. Demgemäß ist es sinnvoll, wenn die Aussparungen durch eine in einer Innenumfangswand der Aufnahmehülse/Tulpe ausgesparte Hinterschneidung, welche vorzugsweise rechteckig, weiter vorzugsweise quadratisch ist, ausgebildet sind, um die Fortsätze aufnehmen zu können. Grundsätzlich sind auch andere Ausgestaltungen möglich. Bei einer quadratischen Hinterschneidung ist jedoch ggf. von Vorteil, dass die Hülse auch um 90° verdreht montiert werden kann, um die Beweglichkeit des Schraubenkopfs in der gleichen Aufnahmehülse/Tulpe in unterschiedlichen (Schwenk-) Richtungen zu ermöglichen.

Insbesondere stützen sich die Fortsätze in der durch die Aussparungen in der Aufnahmehülse oder Tulpe gebildete Hinterschneidung sowohl schraubendiametral nach außen als auch in Schraubenlängsrichtung ab. In anderen Worten ausgedrückt sind die Aussparungen derart in eine Innenumfangsfläche der Aufnahmehülse/Tulpe eingelassen, dass diese sowohl in schraubendiametraler als auch in der Schraubenlängsrichtung Anschläge oder Stützflächen ausbilden. Demgemäß wird nach dieser Ausgestaltung durch die Aussparungen neben der schraubendiametralen Führung oder Abstützung der Fortsätze des Schraubenkopfs auch deren Führung oder Abstützung in der Schraubenlängsrichtung übernommen. Das heißt, sämtliche Relativbewegungen des Schraubenkopfs werden sowohl über die Einsetzhülse (Inlay/Insert) als auch über die Aufnahmehülse/Tulpe geführt oder gestützt und die zu übertragenen Lasten werden aufgeteilt. Entsprechend wird die Steifigkeit der Abstützung des Schraubenkopfs bei Querlasten weiter erhöht.

Vorzugsweise sind die Fortsätze jeweils schraubenradial innen flach ausgebildet und werden an gegenüberliegenden Flächen der Rücksprünge geführt. Das heißt, die Fortsätze bilden bei dieser Ausgestaltung sowohl die radial oder schraubendiametral nach außen gerichteten Anschläge als auch radial oder schraubendiametral nach innen gerichtete Führungsflächen aus. Diese können ggf. zusätzlich Stützfunktionen übernehmen. Die Fortsätze sind folglich derart gestaltet, dass sie sowohl die Vorteile der halbmondförmigen Fortsätze also auch die Vorteile der vorstehend beschriebenen Stützlaschen bereitstellen. Dadurch kann die Steifigkeit der Abstützung des Schraubenkopfs bei Querlasten weiter erhöht werden.

Alternativ oder zusätzlich kann bei jeder der vorstehend beschriebenen Ausgestaltungen der Erfindung ein Teil des Schraubenkopfs zylindrische abgefräst sein, um an dem Schraubenkopf eine weitere Lagerfläche bereitzustellen, welche mit korrespondierenden, stirnseitig an der Einsetzhülse bzw. dem Insert/Inlay ausgebildeten Lagerflächen als Gleitlager zusammenwirkt.

Zusammenfassend kann gesagt werden, dass die der Erfindung zugrunde liegende Aufgabe durch eine Anbringung von seitlichen Laschen am Kopfende einer Knochen-/Pedikelschraube und dazu korrespondierenden Innenräumen an einer Aufnahmehülse bzw. an einem Body bzw. einer Tulpe, sowie durch gleichzeitig korrespondierende Aussparungen am Insert. Die Laschen stützten sich sowohl am Insert als auch am Body in lateraler Richtung ab und sperren eine Bewegung in lateraler Richtung bei gleichzeitig freier Beweglichkeit in kranial-kaudaler Richtung. Dabei bilden der Schraubenkopf und die Laschen eine Einheit und weisen außen eine zylindrische Kontur auf. Durch die erfindungsgemäße Gestaltung sind keine zusätzlichen Verbindungselemente wie Stifte, Achsen und ähnliches notwendig. Zur Montage der gesamten Pedikelschraube (nach einer Ausgestaltung, siehe die nachfolgend beschrieben dritte Ausführungsform) kann die Knochenschraube trotz eines Außendurchmessers der Laschen, der größer ist als der Innendurchmesser des Bodys, mittels einer Drehbewegung der Schraube gegenüber dem Body ohne Werkzeug montiert werden. Aufgrund des größeren Laschendurchmessers stützt sich die Schraube bei Verwendung nicht nur radial am Body ab, sondern auch axial über die Stirnseiten der Laschen.

### Kurzbeschreibung der Figuren

Nachfolgend wird die vorliegende Erfindung anhand bevorzugter Ausführungsformen beschrieben, welche nachfolgend veranschaulichend beschrieben werden. Es ist anzumerken, dass Merkmale verschiedener Ausführungsformen kombinierbar sind und diverse Änderungen vorgenommen werden können, ohne vom Schutzumfang der Erfindung abzuweichen.
Fig. 1 zeigt eine perspektivische Teilschnittansicht der Pedikelschraube nach einer ersten Ausführungsform der Erfindung;
Fig. 2 zeigt eine Aufnahmehülse oder Tulpe im Längsschnitt nach der ersten Ausführungsform;
Fig. 3 zeigt einen Schraubenkopf nach einer zweiten Ausführungsform der Erfindung;
Fig. 4 zeigt eine Einsetzhülse nach der zweiten Ausführungsform der Erfindung;
Fig. 5 ist eine Längsschnittansicht der zusammengebauten Pedikelschraube nach der zweiten Ausführungsform der Erfindung;
Fig. 6 zeigt einen Querschnitt der Aufnahmehülse nach der zweiten Ausführungsform im Bereich der Aussparungen;
Fig. 7 ist eine Seitenansicht eines Schraubenkopfs nach einer dritten Ausführungsform der Erfindung;
Fig. 8 zeigt eine Einsetzhülse nach der dritten Ausführungsform der Erfindung;
Fig. 9 zeigt eine perspektivische Teilschnittansicht der Pedikelschraube nach der dritten Ausführungsform; und
Fig. 10 zeigt eine Längsschnittansicht der Pedikelschraube nach der dritten Ausführungsform der Erfindung.

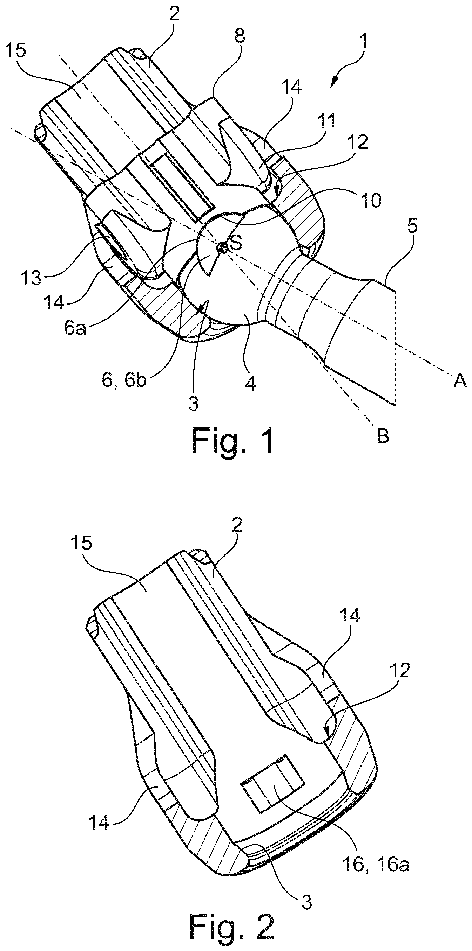

Fig. 1 zeigt eine perspektivische Teilschnittansicht einer erfindungsgemäßen Pedikelschraube 1 in einem abgewinkelten Zustand. Die Pedikelschraube 1 weist eine in dieser Darstellung längsgeschnittene Tulpe oder Aufnahmehülse 2 auf, deren Innenumfang an einem distalen Ende konisch verengt ist, um eine Anlagefläche 3 für einen kugeligen Schraubenkopf 4 der Pedikelschraube 1 zu bilden. Die Pedikelschraube 1 wurde aus proximaler Richtung derart in die Aufnahmehülse 2 eingeführt, dass der kugelige Schraubenkopf 4 distal an der Anlagefläche 3 anliegt und ein damit integral ausgebildeter Schraubenschaft 5 distal aus der Aufnahmehülse 2 herausragt, sodass der Schraubenkopf 4 und Schraubenschaft 5 relativ zu der Aufnahmehülse 2 verschwenkbar sind.

Der Schraubenkopf 4 ist auch proximal zumindest teilweise kugelig ausgebildet, und weist ferner an einander schraubendiametral gegenüberliegenden Seiten zwei Fortsätze 6 auf, welche sich ausgehend von einem Außenumfang des Schraubenkopfs 4 halbmondförmig in proximaler Richtung erstrecken. D. h., proximal ausgerichtete Flächen der Fortsätze 6 bildet jeweils Lagerflächen 6a, welche zum Abstützen des Schraubenkopfs 4 an einer Einsetzhülse 8 (Inlay/Insert) dienen. Diese Lagerflächen 6a bzw. deren Durchmesser definiert bei der vorliegenden Pedikelschraube 1 eine Schwenkachse S, um welche der Schraubenkopf 4 bezüglich der Aufnahmehülse 2 verschwenkbar ist. Um dies zu veranschaulichen ist die Pedikelschraube 1 in Fig. 1 in einem verschwenkten Zustand dargestellt, was durch die Längsachsen des Schraubenschafts A und der Aufnahmehülse B deutlich wird, welche sich an einer Schwenkachse, d. h. der Mittelachse der Lagerflächen 6a, schneiden. Darüber hinaus bilden die Fortsätze 6 eine vorzugsweise teilzylinderförmige radiale Außenfläche oder einen vorzugsweise teilzylinderförmigen radialen Anschlag 6b, die/der als eine Stützfläche zum Abstützen des Schraubenkopfs 4 an der Aufnahmehülse 2 dienen.

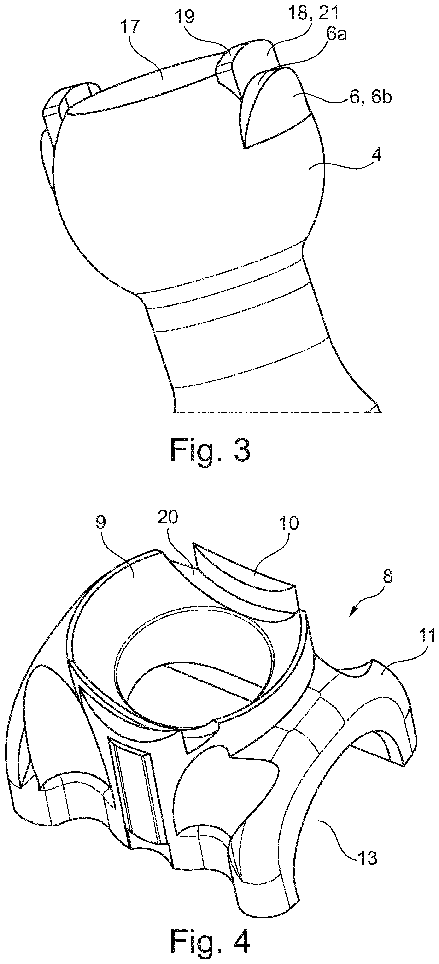

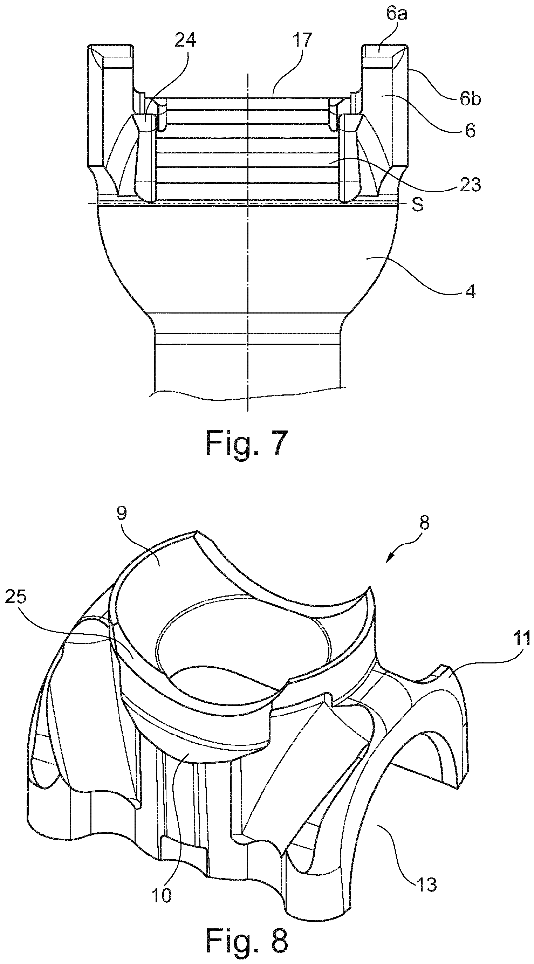

Die Einsetzhülse 8 bildet an ihrer distalen Seite zur Aufnahme des runden Schraubenkopfs 4 teilweise eine hohlkugelförmige Aufnahmefläche 9 aus, welche hier nicht dargestellt ist, aber im Wesentlichen der korrespondierenden Aufnahmefläche 9 aus Fig. 4 und Fig. 8 entspricht. Ferner ist ein stirnseitiger, distaler Rand der Einsetzhülse 8 nicht durchgehend ausgebildet, sondern weist zwei einander diametral gegenüberliegende Rücksprünge 10 auf, welche mit dem halbmondförmigen oder ohrenartigen Fortsätzen 6, genauer, deren Lagerflächen 6a, korrespondieren. Die Rücksprünge 10 wirken mit den Lagerflächen 6a nach Art eines Gleitlagers zusammen, um die Verschwenkbarkeit der Aufnahmehülse 2 und des Schraubenkopfs 4 zu gewährleisten.

An einem proximalen Bereich der Einsetzhülse 8 weist diese zwei zu den Rücksprüngen 10 um 90° versetzte laterale Ausbuchtungen 11 auf, welche in korrespondierende Sattelflächen 12 innerhalb der Aufnahmehülse 2 lagebestimmt aufgenommen sind. Die Einsetzhülse 8 hat ferner eine halbzylindrische Öffnung 13, welche durch die Ausbuchtungen 11 verläuft, um eine Verbindungstange aufzunehmen, über welche bei einer Implantation oder Operation zwei Pedikelschrauben 1 miteinander verbunden werden können. Die Aufnahmehülse 2 hat ferner an der gleichen Winkelposition wie die Sattelflächen 12 zwei von proximaler Seite aus eingebrachte, U-förmige Schlitze 14, in welche die Verbindungstange einlegbar ist. An einem proximalen Bereich innerhalb der Aufnahmehülse 2 ist ein Gewinde 15 vorgesehen, in welches eine Madenschraube zum Verspannen der Pedikelschraube 1 und der Verbindungstange eingeschraubt werden kann.

Fig. 2 zeigt einen Längsschnitt der Aufnahmehülse 2 nach der ersten Ausführungsform der Erfindung. Neben den bereits vorstehend beschriebenen Merkmalen geht aus dieser Ansicht hervor, dass in einem Innenumfangsbereich der Aufnahmehülse 2, in welchem der Schraubenkopf 4 aufgenommen ist, 2 einander diametral gegenüberliegende Aussparungen 16 (hier nur eine dargestellt) vorgesehen sind. Diese Aussparungen 16 dienen dazu, die Fortsätze 6 aufzunehmen, um die Beweglichkeit oder Verschwenkbarkeit des Schraubenkopfs um die Schwenkachse S zu gewährleisten. Gleichzeitig bilden die Aussparungen 16 eine Stütz- oder Anschlagsfläche 16a, um dem Schraubenkopf 4 über die Fortsätze 6, genauer, die radialen Anschläge 6b, unmittelbar an der Aufnahmehülse 2 abzustützen.

Die in Fig. 3 bis Fig. 6 dargestellte zweite Ausführungsform der Erfindung entspricht zu großen Teilen der der ersten Ausführungsform, weshalb nachfolgend lediglich auf deren Unterschiede eingegangen wird und dieselben Bezugszeichen vergleiche Merkmale verwendet werden.

Fig. 3 zeigt dem Schraubenkopf 4 nach der zweiten Ausführungsform der Erfindung in einer perspektivischen Ansicht. Hier ist zu sehen, dass der Schraubenkopf 4 eine proximale Stirnfläche 17 aufweist, in welcher beispielsweise eine Werkzeugaufnahme oder ein Kanal zum Einpressen von Knochenzement eingelassen sein kann. Eine solche Stirnfläche 17 kann auch bei der ersten Ausführungsform vorgesehen sein. Ferner weist der Schraubenkopf 4 zusätzlich zu den Fortsätzen 6 dazu radial nach innen versetzte Stützlaschen 18 auf. Diese Stützlaschen 18 erstrecken sich in proximaler Richtung über die proximale Stirnfläche 17 des Schraubenkopfs 4 hinaus und bilden an ihrem proximalen Ende ebenfalls Zylindermantelflächen oder zweite Lagerflächen 19 aus, um eine weitere Führungs- oder Stütz- oder Lagerfläche zum Führen oder Stützen oder Lagern an der Einsetzhülse 8 zu bilden. In schraubendiametraler Richtung sind die Stützlaschen 18 beidseitig flach ausgebildet.

Fig. 4 zeigt eine Einsetzhülse 8 nach der zweiten Ausführungsform der Erfindung. Diese weist neben der Aufnahmefläche 9 und den Rücksprüngen 10 distal eingelassene Schlitze 20 auf, welche zu den Rücksprüngen 10 radial nach innen versetzt sind. D. h., eine Position der Schlitze 20 korrespondiert zu einer Position der Stützlaschen 18. Demgemäß bilden die Schlitze 20 in distaler Richtung eine innenzylindrische Fläche aus, welche mit dem Stützlaschen 18, genauer, der Zylindermantelfläche oder der zweiten Lagerfläche 19 der Stützlaschen 18, nach Art eines Gleitlagers zusammenwirken.

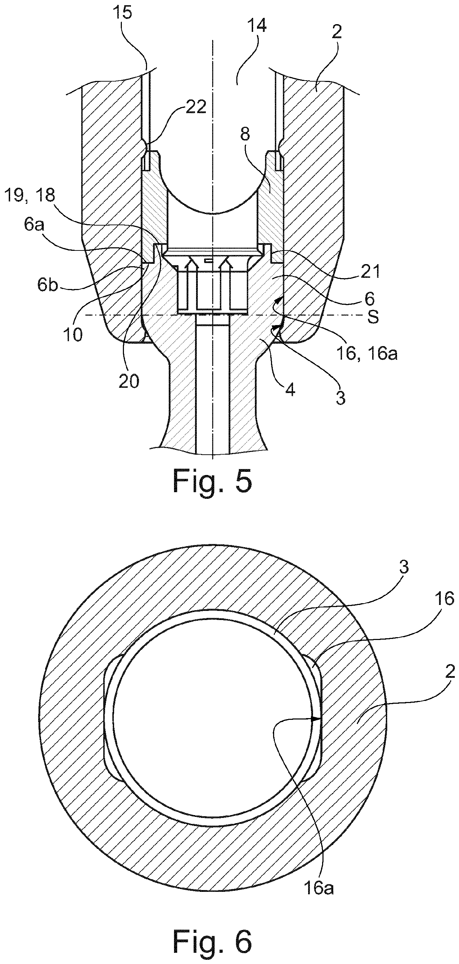

Fig. 5 zeigt eine zusammengebaute Pedikelschraube 1 nach der zweiten Ausführungsform im Längsschnitt. Darin ist erkennbar, wie die Fortsätze 6 des Schraubenkopfs 4 schraubendiametral an der Aufnahmehülse 2, genauer, an den Stütz- oder Anschlagsflächen 16a der Aussparungen 16, anliegen, um sich in schraubendiametrale Richtung abzustützen. Ferner ist erkennbar, dass die Lagerflächen 6a der Fortsätze 6 des Schraubenkopfs 4 und die Rücksprünge 10 der Einsetzhülse 8 sowie die zweite Lagerflächen 19 der Stützlaschen 18 und die Schlitze 20 der Einsetzhülse 8 aneinander anliegen, um den Schraubenkopf 4 in Schraubenlängsrichtung an der Einsetzhülse 8 abzustützen und führen. Darüber hinaus ist erkennbar, dass die Stützlaschen 18 sich zumindest mit dem radial äußeren flachen Seiten 21 an korrespondierenden Seitenwänden der Schlitze 20 abstützen, um die Stabilität der Pedikelschraube 1 gegen ein Verschwenken in einer Richtung quer zu der vorgesehenen Schwenkachse S (Querverschwenken) zu erhöhen. In der Aufnahmehülse 2 sind ferner nach dieser Ausführungsform 2 einander diametral gegenüberliegende, um 90° zu den Schlitzen 14 versetzte Stoppvorsprünge 22 bereitgestellt, welche dazu dienen, die Einsetzhülse 8 provisorisch (d. h., solange die Verbindungstange noch nicht eingesetzt und die Madenschraube noch nicht eingeschraubt ist) in der Aufnahmehülse 2 zu halten.

Fig. 6 zeigt einen Querschnitt der Aufnahmehülse 2 in dem Bereich, in welchem der Schraubenkopf 4 aufgenommen wird. Insbesondere geht aus dieser Darstellung die Form der Aussparungen 16 hervor, in welchem die Fortsätze 6 des Schraubenkopfs 4 schwenkbar aufgenommen sind. Die Aussparungen 16 sind im Querschnitt im Wesentlichen rechteckig, wodurch die radialen Anschläge 6b der Fortsätze 6 des Schraubenkopfs in jeder Schwenkposition an der durch die Aussparungen 16 ausgebildeten Stütz- oder Anschlagsflächen 16a anliegt. Auch die Aussparungen 16 in der ersten Ausführungsform weisen eine solche Form auf, können jedoch kleiner ausgebildet sein.

Fig. 7 zeigt eine Seitenansicht eines Schraubenkopfs 4 nach einer dritten Ausführungsform der Erfindung. Die Pedikelschraube 1 nach dieser Ausführungsform ist verglichen mit der Pedikelschraube nach der ersten und zweiten Ausführungsform dazu ausgebildet, besonders hohe Kräfte zu übertragen, und ist deshalb besonders stabil ausgebildet. Nachfolgend wird auf die Besonderheiten und Unterschiede dieser Ausführungsform gegenüber den anderen beiden Ausführungsformen, insbesondere der ersten, eingegangen, wobei dieselben Bezugszeichen vergleiche Merkmale verwendet werden.

Auch dieser Schraubenkopf 4 ist im Wesentlichen kugelförmig/sphärisch und bildet an eine proximale Stirnfläche 17, in welcher gegebenenfalls eine Werkzeugaufnahme oder ein Kanal eingelassen sein können. Ferner ist eine proximale Hälfte der Kugeloberfläche des Schraubenkopfs 4 mit einer Riffelung oder Rippen 23 versehen. Durch diese ist der Schraubenkopf 4 gegenüber der Einsetzhülse 8 nicht frei um die Schwenkachse S verschwenkbar, sondern kann über die Rippen 23 in einer bestimmten Stellung oder Schwenkposition eingerastet werden. Ferner sind die Fortsätze 6 sehr groß ausgebildet. D. h., sie ragen in schraubendiametraler Richtung über dem Außenumfang des sphärischen Grundkörpers des Schraubenkopfs 4 hinaus, sodass eine schraubendiametrale Erstreckung der Fortsätze 6 größer als der Durchmesser des sphärischen Grundkörpers des Schraubenkopfs 4 ist. Zudem ragen die Fortsätze 6 in Schraubenlängsrichtung über die proximale Stirnfläche 17 des Schraubenkopfs 4 hinaus. Auf diese Weise können die Fortsätze 6 an ihrer radialen Innenseite zusätzliche Stützflächen ausbilden, welche sich ähnlich den Stützlaschen 18 der zweiten Ausführungsform an korrespondierenden Flächen der Einsetzhülse 8 abstützen, um eine zusätzliche Stabilität der Pedikelschraube gegen eine quer Verschwenkung zu erreichen. Ggf. können die Fortsätze 6 auch dazu dienen, die Einsetzhülse 8 dazwischen einzuspannen/einzuklemmen.

Radial innerhalb der Fortsätze 6 ist der kugelige Schraubenkopf 4 zylindrisch ausgespart, z.B. durch Fräsen, um eine Schwenkbewegung der Aufnahmehülse 2 zwischen den Fortsätzen 6 des Schraubenkopfs 4 nicht zu behindern. Gegebenenfalls kann dadurch zusätzlich zu dem durch die Fortsätze 6 gebildeten Lagerflächen 6a eine weitere Lagerfläche 24 bereitgestellt sein. Zusammenfassend kann man sagen, dass die dritte Ausführungsform im Wesentlichen eine Kombination der ersten und zweiten Ausführungsform darstellt, welche eine besonders hohe Stabilität gegen eine Querverschwenkung ermöglicht.

Fig. 8 zeigt eine perspektivische Ansicht der Einsetzhülse 8 nach der dritten Ausführungsform der Erfindung. Darin ist gut erkennbar, dass die Rücksprüngen 10 der Einsetzhülse 8 deutlich tiefer als die der ersten und zweiten Ausführungsform sind, damit die großen Fortsätze 6 darin Platz haben. Ferner sind die Rücksprünge 10 radial nach außen versetzt, wodurch jeweils eine Stufe entsteht. Dabei ist auch der radial innenliegende, weiter distal liegende Teil von jeder Stufe zylindrisch ausgespart, um eine Schwenkbewegung der Aufnahmehülse 2 zwischen den Fortsätzen 6 des Schraubenkopfs 4 nicht zu behindern. Zudem können gegebenenfalls durch diese weiter distal liegenden Teile der Stufen zusätzliche Lagerflächen 25 ausgebildet werden, welche dazu angepasst sind, mit dem Lagerflächen 24 des Schraubenkopfs 4 nach Art eines Gleitlagers zusammenzuwirken.

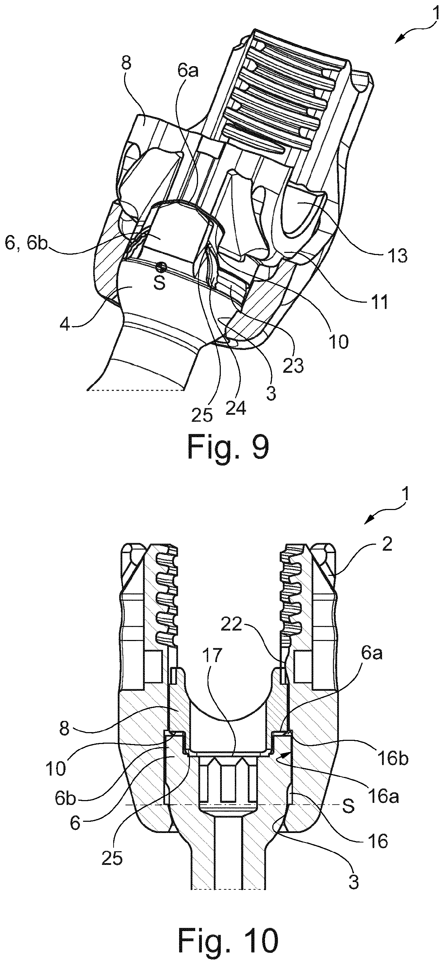

Fig. 9 zeigt eine perspektivische Teil Schnittansicht der zusammengebauten Pedikelschraube 1 nach der dritten Ausführungsform der Erfindung. Darin ist gut zu erkennen, wie die Aufnahmefläche 9 der Einsetzhülse 8 auf den Rippen 23 des Schraubenkopfs 4 sitzt und wie die Fortsätze 6 in die Rücksprüngen 10 der Einsetzhülse 8 greifen, um sowohl über die Lagerflächen 6a der Fortsätze 6 mit den Rücksprüngen 10 der Einsetzhülse 8 in Schraubenlängsrichtung als auch über die radial innenliegenden Stützflächen der Fortsätze 6 mit den durch die Rücksprüngen 10 ausgebildeten Stufen in schraubendiametrale Richtung Wirkflächenpaare auszubilden.

Fig. 10 zeigt eine zusammengebaute Pedikelschraube nach der dritten Ausführungsform im Längsschnitt, woraus eine weitere Besonderheit der vorliegenden Ausführungsform hervorgeht. Und zwar ist erkennbar, dass die Aussparungen 16 in der Aufnahmehülse 2 eine Hinterschneidung bilden. D. h., durch die Aussparungen 16 wird nicht nur die schraubendiametral ausgerichtete Stütz- oder Anschlagsfläche 16a, sondern auch eine proximale, schraubenlängs ausgerichtete Zusatzstützfläche 16b bereitgestellt, an welcher die Lagerflächen 6a der Fortsätze 6 des Schraubenkopfs 4 in Anlage kommt. Auf diese Weise wird gewährleistet, dass der Schraubenkopf 4 sich auch in Schraubenlängsrichtung nicht nur an der Einsetzhülse 8 sondern auch unmittelbar an der Aufnahmehülse 2 abstützt. D. h., nach dieser Ausführungsform wird der Schraubenkopf 4 sowohl in proximaler als auch in schraubendiametrale Richtung unmittelbar durch die Aufnahmehülse 2 und die Einsetzhülse 8 gestützt und wird eine maximale Stabilität gegen eine Querverschwenkung erreicht. Die Aussparung 16 kann im Querschnitt quadratisch sein, sodass der Schraubenkopf grundsätzlich auch quer eingesetzt werden kann und die gleiche Aufnahmehülse für verschieden ausgerichtete polyaxiale Pedikelschrauben verwendbar ist.

### Bezugszeichenliste

- 1: Pedikelschraube
- 2: Aufnahmehülse/Tulpe
- 3: Anlagefläche der Aufnahmehülse
- 4: Schraubenkopf
- 5: Schraubenschaft
- 6: Fortsatz des Schraubenkopfs
- 6a: Lagerflächen des Schraubenkopfs
- 6b: diametrale Anschläge des Schraubenkopfs
- 7 8: Einsetzhülse/Insert/Inlay
- 9: Aufnahmefläche der Einsetzhülse
- 10: Rücksprung der Einsetzhülse
- 11: Ausbuchtung Einsetzhülse
- 12: Sattelfläche der Aufnahmehülse
- 13: halb zylindrische Öffnung der Einsetzhülse
- 14: U-förmige Schlitz der Aufnahmehülse
- 15: Gewinde der Aufnahmehülse
- 16: Aussparung der Aufnahmehülse
- 16a: schraubendiametrale Stütz- oder Anschlagsfläche der Aussparung
- 16b: proximale Stützfläche der Aussparung
- 17: proximale Stirnfläche des Schraubenkopfs
- 18: Stützlasche des Schraubenkopfs
- 19: zweite Lagerfläche der Stützlasche
- 20: Schlitz der Einsetzhülse
- 21: Flache Seiten der Stützlasche
- 22: Stoppvorsprung der Aufnahmehülse
- 23: Rippen des Schraubenkopfs
- 24: Lagerfläche (oder Aussparung) des Schraubenkopfs
- 25: Lagerfläche (oder Stufe) der Einsetzhülse

## Patentansprüche

1. Pedikelschraube (1) der Polyaxialbauart mit einem Schraubenschaft (5), an dessen proximalen Ende ein Schraubenkopf (4) mit im Wesentlichen sphärischer Grundform einstückig ausgebildet ist, der in einer Aufnahmehülse oder Tulpe (2) gelagert ist, in der eine Einsetzhülse (8) eingesetzt ist, die zur Festlegung einer Relativ-Schwenkposition zwischen der Aufnahmehülse oder Tulpe (2) und dem Schraubenschaft (5) gegen den Schraubenkopf (4) andrückbar ist,
wobei der Schraubenkopf zwei einander diametral gegenüberliegende Schwenkführungs- oder Schwenkbeschränkungseinheiten (127) aufweist, **dadurch gekennzeichnet, dass** die zwei einander diametral gegenüberliegenden Schwenkführungs- oder Schwenkbeschränkungseinheiten jeweils zumindest einen radial sowie in Schraubenlängsrichtung über die sphärische Grundform des Schraubenkopfs (4) hinausragenden Fortsatz (6) aufweisen, wobei die Fortsätze (6) an ihren jeweiligen, vorzugsweise teilzylinderförmigen, radialen Außenflächen Anschläge (6b) bilden und derart positioniert sind, dass sie eine Relativ-Verschwenkung des Schraubenschafts (5) und der Tulpe oder Aufnahmehülse (2) nur in einer Schwenkebene erlauben und dass sie bei einer Relativ-Verschwenkung in einer anderen Schwenkebene gegen eine radial innere Umfangsseite der Tulpe oder Aufnahmehülse (2) stützend anschlagen.

2. Pedikelschraube (1) nach Anspruch 1, wobei die Fortsätze (6) proximal teilweise zylinderförmige Lagerflächen (6a) bilden, welche die Relativ-Verschwenkung des Schraubenschafts (5) und des Tulpe oder Aufnahmehülse (2) in der Schwenkebene führen und welche in proximaler Richtung an stirnseitigen oder distalen, entsprechend teilzylindrisch ausgebildeten Rücksprüngen (10) der Einsetzhülse (8) geführt sind, um nach Art eines Gleitlagers zusammenzuwirken.

3. Pedikelschraube (1) nach einem der Ansprüche 1 und 2, wobei die Tulpe oder Aufnahmehülse (2) an einer Innenumfangsfläche zumindest zwei einander diametral gegenüberliegende Aussparungen (16) aufweist, welche ausreichend groß dimensioniert sind, dass die Fortsätze (6) in jeder Schwenkposition darin aufgenommen sind.

4. Pedikelschraube (1) nach einem der vorstehenden Ansprüche, wobei eine Verschwenkung des Schraubenschafts (5) relativ zu der Aufnahmehülse oder Tulpe (2) in der einen Schwenkebene von zumindest +/- 22°, vorzugsweise zumindest +/- 30°, zugelassen wird.

5. Pedikelschraube (1) nach einem der vorstehenden Ansprüche, wobei die Aufnahmehülse oder Tulpe (2) in Umfangsrichtung um 90° zu den Fortsätzen (6) versetzt vorzugsweise U-förmige Schlitze (14) zur Aufnahme einer Verbindungsstange aufweist, durch welche mehrere Pedikelschrauben (1) miteinander verbindbar sind.

6. Pedikelschraube (1) nach einem der Ansprüche 2 bis 5, **dadurch gekennzeichnet, dass** ein Durchmesser der proximalen Lagerflächen (6a) der Fortsätze (6) kleiner als ein Durchmesser der sphärische Grundform des Schraubenkopfs (4) ist.

7. Pedikelschraube (1) nach einem der vorstehenden Ansprüche, wobei der Schraubenkopf (4) zusätzliche Stützlaschen (18) aufweist, welche zu den Fortsätzen (6) schraubendiametral nach innen versetzt sind und welche in proximaler Richtung über die Fortsätze (6) hinausragen.

8. Pedikelschraube (1) nach Anspruch 7, wobei die Stützlaschen (18) proximal teilweise zylinderförmige zweite Lagerflächen (19) bilden, um mit stirnseitigen, entsprechend teilzylindrisch ausgebildeten Schlitzen (20) der Einsetzhülse (8) nach Art eines Gleitlagers zusammenzuwirken.

9. Pedikelschraube (1) nach einem der Ansprüche 7 und 8, wobei die Stützlaschen (18) schraubenradial außen und vorzugsweise innen flach sind, um sich an korrespondierenden Seitenflächen der Schlitze (20) zu stützen, um eine Verschwenkung in der anderen Schwenkebene zu blockieren.

10. Pedikelschraube (1) nach einem der vorstehenden Ansprüche, wobei die Fortsätze (6) schraubenradial nach außen und vorzugsweise proximal über einen Außendurchmesser des sphärischen Grundkörpers des Schraubenkopfs (4) hinausragen.

11. Pedikelschraube (1) nach Anspruch 10, wobei die Aussparungen (16) durch eine in einer Innenumfangswand der Aufnahmehülse oder Tulpe (2) ausgesparte, vorzugsweise rechteckige Hinterschneidung ausgebildet sind.

12. Pedikelschraube (1) nach Anspruch 11, wobei die Fortsätze (6) sich in der durch die Aussparungen (16) in der Aufnahmehülse oder Tulpe (2) gebildete Hinterschneidung sowohl schraubendiametral nach außen als auch in Schraubenlängsrichtung abstützen.

13. Pedikelschraube (1) nach einem der Ansprüche 10 bis 12, wobei die Fortsätze (6) jeweils schraubenradial innen flach ausgebildet sind und an korrespondierenden Flächen der Rücksprünge (10) geführt werden.

## Claims

1. A pedicle screw (1) of the polyaxial type with a screw shaft (5), at the proximal end of which a screw head (4) with a substantially spherical basic shape is formed in one piece, which is mounted in a receiving sleeve or tulip (2), in which an insertion sleeve (8) is inserted, which can be pressed against the screw head (4) to fix a relative pivot position between the receiving sleeve or tulip (2) and the screw shaft (5),
wherein the screw head has two pivot guide units or pivot restriction units (127) that are diametrically opposite to one another, **characterized in that** the two pivot guide units or pivot restriction units (127) that are diametrically opposite to one another respectively have at least one extension (6) projecting radially and in a longitudinal screw direction beyond the spherical basic shape of the screw head (4), wherein the extensions (6) form stops (6b) on their respective, preferably partially cylindrical, radial outer surfaces, and are positioned in such a way that they permit relative pivoting of the screw shaft (5) and the tulip or receiving sleeve (2) only in one pivot plane and that, in the event of relative pivoting in another pivot plane, they are supported to be stopped at a radially inner circumferential side of the tulip or receiving sleeve (2).

2. The pedicle screw (1) according to claim 1, wherein the extensions (6) proximally form partially cylindrical bearing surfaces (6a) which guide the relative pivoting of the screw shaft (5) and the tulip or receiving sleeve (2) in the pivot plane and which are guided in a proximal direction on frontal or distal, correspondingly partially cylindrical recesses (10) of the insertion sleeve (8) in order to interact in the manner of a plain bearing.

3. The pedicle screw (1) according to one of claims 1 and 2, wherein the tulip or receiving sleeve (2) has, on an inner circumferential surface, at least two diametrically opposite cavities (16), which are sufficiently large to accommodate the extensions (6) in any pivoted position.

4. The pedicle screw (1) according to one of the preceding claims, wherein pivoting of the screw shaft (5) relative to the receiving sleeve or tulip (2) in the one pivot plane is permitted by at least +/- 22°, preferably at least +/- 30°.

5. The pedicle screw (1) according to one of the preceding claims, wherein the receiving sleeve or tulip (2) has, offset in the circumferential direction by 90° with respect to the extensions (6), preferably U-shaped slots (14) for receiving a connecting rod, via which a plurality of pedicle screws (1) can be connected to each other.

6. The pedicle screw (1) according to one of claims 2 to 5, **characterized in that** a diameter of the proximal bearing surfaces (6a) of the extensions (6) is smaller than a diameter of the basic spherical shape of the screw head (4).

7. The pedicle screw (1) according to one of the preceding claims, wherein the screw head (4) has additional support lugs (18) which are screw-diametrically offset inwards with respect to the extensions (6) and which project beyond the extensions (6) in the proximal direction.

8. The pedicle screw (1) according to claim 7, wherein the support lugs (18) form proximally partially cylindrical second bearing surfaces (19) in order to interact with frontal, correspondingly partially cylindrical slots (20) of the insertion sleeve (8) in the manner of a plain bearing.

9. The pedicle screw (1) according to one of claims 7 and 8, wherein the support lugs (18) are screw-radially flat on the outside and preferably on the inside in order to support themselves on corresponding side surfaces of the slots (20) to block pivoting in the other pivot plane.

10. The pedicle screw (1) according to one of the preceding claims, wherein the extensions (6) project screw-radially outwards and preferably proximally beyond an outer diameter of the spherical base body of the screw head (4).

11. The pedicle screw (1) according to claim 10, wherein the cavities (16) are formed by a preferably rectangular undercut recessed in an inner circumferential wall of the receiving sleeve or tulip (2).

12. The pedicle screw (1) according to claim 11, wherein the extensions (6) are supported in the undercut formed by the cavities (16) in the receiving sleeve or tulip (2) both screw-diametrically outwards and in the longitudinal screw direction.

13. The pedicle screw (1) according to one of claims 10 to 12, wherein the extensions (6) are each flat screw-radially on the inside and are guided on corresponding surfaces of the recesses (10).

## Revendications

1. Vis pédiculaire (1) de conception polyaxiale avec une tige de vis (5), à l'extrémité proximale de laquelle une tête de vis (4) est formée d'un seul tenant avec une forme de base sensiblement sphérique qui est logée dans une douille de réception ou tulipe (2), dans laquelle une douille d'insertion (8) est insérée, douille qui peut être pressée pour la définition d'une position de pivotement relative entre la douille de réception ou tulipe (2) et la tige de vis (5) contre la tête de vis (4),
dans laquelle la tête de vis présente deux unités de guidage de pivotement ou de limitation de pivotement diamétralement opposées l'une à l'autre, **caractérisée en ce que** les deux unités de guidage de pivotement ou de limitation de pivotement diamétralement opposées l'une à l'autre présentent respectivement au moins un prolongement (6) dépassant radialement ainsi que dans le sens longitudinal de la vis au-delà de la forme de base sphérique de la tête de vis (4), dans laquelle les prolongements (6) forment des butées (6b) au niveau de leurs surfaces extérieures radiales respectives, de préférence en forme de cylindre partiel, et sont positionnés de telle manière qu'ils permettent un pivotement relatif de la tige de vis (5) et de la tulipe ou douille de réception (2) uniquement dans un plan de pivotement et **en ce qu'**ils viennent en butée contre un côté périphérique radialement intérieur de la tulipe ou douille de réception (2) lors d'un pivotement relatif dans un autre plan de pivotement.

2. Vis pédiculaire (1) selon la revendication 1, dans laquelle les prolongements (6) forment des surfaces de palier (6a) partiellement cylindriques de manière proximale qui entraînent le pivotement relatif de la tête de vis (5) et de la tulipe ou douille de réception (2) dans le plan de pivotement et qui sont guidées en direction proximale au niveau de retraits (10) partiellement cylindriques avant ou distaux de la douille d'insertion (8) afin de coopérer à la manière d'un palier lisse.

3. Vis pédiculaire (1) selon l'une quelconque des revendications 1 et 2, dans laquelle la tulipe ou douille de réception (2) présente au niveau d'une surface périphérique intérieure au moins deux évidements (16) opposés diamétralement l'un à l'autre qui sont dimensionnés de manière suffisamment grande que les prolongements (6) soient reçus à l'intérieur dans chaque position de pivotement.

4. Vis pédiculaire (1) selon l'une quelconque des revendications précédentes, dans laquelle un pivotement de la tige de vis (5) par rapport à la douille de réception ou tulipe (2) est autorisé dans l'un plan de pivotement d'au moins +/- 22°, de préférence d'au moins +/- 30°.

5. Vis pédiculaire (1) selon l'une quelconque des revendications précédentes, dans laquelle la douille de réception ou tulipe (2) déportée de 90° dans le sens périphérique par rapport aux prolongements (6) présente des fentes (14) de préférence en forme de U pour la réception d'une tige de liaison, via laquelle plusieurs vis pédiculaires (1) peuvent être reliées entre elles.

6. Vis pédiculaire (1) selon l'une quelconque des revendications 2 à 5, **caractérisée en ce qu'**un diamètre des surfaces de palier (6a) proximales des prolongements (6) est inférieur à un diamètre de la forme de base sphérique de la tête de vis (4).

7. Vis pédiculaire (1) selon l'une quelconque des revendications précédentes, dans laquelle la tête de vis (4) présente des languettes d'appui (18) supplémentaires qui sont en déport vers l'intérieur de manière diamétrale à la vis par rapport aux prolongements (6) et qui font saillie en direction proximale au-delà des prolongements (6).

8. Vis pédiculaire (1) selon la revendication 7, dans laquelle les languettes d'appui (18) forment de secondes surfaces de palier (19) partiellement cylindriques de manière proximale afin d'interagir avec des fentes (20) partiellement cylindriques du côté avant de la douille d'insertion (8) à la manière d'un palier lisse.

9. Vis pédiculaire (1) selon l'une quelconque des revendications 7 et 8, dans laquelle les languettes d'appui (18) sont plates de manière radiale à la vis à l'extérieur et de préférence à l'intérieur afin d'appuyer contre des surfaces latérales correspondantes des fentes (20) afin de bloquer un pivotement dans l'autre plan de pivotement.

10. Vis pédiculaire (1) selon l'une quelconque des revendications précédentes, dans laquelle les prolongements (6) font saillie de manière radiale à la vis vers l'extérieur et de préférence de manière proximale au-delà d'un diamètre extérieur du corps de base sphérique de la tête de vis (4).

11. Vis pédiculaire (1) selon la revendication 10, dans laquelle les évidements (16) sont réalisés par une contre-dépouille de préférence rectangulaire, évidée dans une paroi périphérique intérieure de la douille de réception ou tulipe (2).

12. Vis pédiculaire (1) selon la revendication 11, dans laquelle les prolongements (6) s'appuient dans la contre-dépouille formée par les évidements (16) dans la douille de réception ou tulipe (2) aussi bien diamétralement à la vis vers l'extérieur que dans le sens longitudinal de la vis.

13. Vis pédiculaire (1) selon l'une quelconque des revendications 10 à 12, dans laquelle les prolongements (6) sont réalisés pour être plats respectivement de manière radiale à la vis à l'intérieur et sont guidés au niveau de surfaces correspondantes des retraits (10).
